# EUROPEAN PATENT SPECIFICATION

(11) **EP 0 962 768 B1**
(45) Date of publication and mention of the grant of the patent: **08.02.2006**
(21) Application number: 99850090.4
(22) Date of filing: 25.05.1999
(51) Int. Cl.: G01N 33/02, G01N 1/38

(54) **Product for dispersing of foodstuffs for analytical purposes**
Produkt zum Dispergieren von Lebensmitteln für analytische Zwecke
Produit pour la dispersion de produits alimentaires à des fins d'analyse

(30) Priority: 25.05.1998 SE 9801827
(43) Date of publication of application: 08.12.1999
(73) Proprietor: L O Sjaunja Aktiebolag, 753 18 Uppsala (SE)
(72) Inventor: Sjaunja, Lars-Ove, 752 42 Uppsala (SE)
(74) Representative: Lindgren, Anders

(56) References cited:
- EP-A- 0 455 923
- WO-A-81/02467
- US-A- 3 573 062
- US-A- 5 554 397
- THOMAS M A ET AL: "Effect of emulsifying salts on objective and subjective properties of processed cheese." JOURNAL OF FOOD SCIENCE 1980 DAIRY RES. CENT., PO BOX 217, RICHMOND, NSW, AUSTRALIA 2753, vol. 45, no. 3, pages 458-459, 466, XP001109708
- OOGHE W ET AL: "COMPARISON OF SOME ADDITIVES USED IN THE PREPARATION OF FREEZE-DRIED LEMON JUICE CANDIDATE REFERENCE MATERIALS" STN CHEMICAL ABSTRAC, XP002951421

## Description

The present invention generally relates to a product for making it possible to dissolve and disperse foodstuffs of different kinds to provide a dispersion, and the invention is more particularly concerned with such a product which is suited for dispersing of foodstuffs solely for analysis for the contents of said foodstuff.

By "foodstuff" in meant, in this connection, all kinds of foodstuffs for human beings or for animals, like the dairy products milk - butter - cheese - cultured milk products etc., other types of edible fat products, chocolate, bone free meat, bone free fish, flour and grain products and most other existing solid foodstuffs like ready prepared food and quick food, feedstuff for animals like cereals, meat, fish as concentrated fodder, dog food and cat food.

Several different methods are known, by means of which the content of the foodstuff can be analysed, especially as concerns the content of fats, proteins, carbohydrates, organic acids, water and eventual other, foreign substances.

Analysis of the contents of the various foodstuffs generally is made by analytic, chemical methods. For said methods it is necessary to perform an extensive sample preparation before the actual component can be detected. It is often necessary that the actual component be isolated from the other components following various treatment steps. In the analysis each component of the product normally has to analysed separately. A chemical analysis of a foodstuff product therefore may consist of at least five different analysis steps. Such method are time consuming and are therefore expensive to perform.

Lately various types of physicalic chemical methods have been introduced in the foodstuff industry. In for instance enzymatic food analysis there are used UVNIS spectroscopic methods, whereby the reactions of specific enzymes can be used for quantification of various substances for instance in the foodstuff. In the enzymatic food analysis, for instance in the Boehringer-Mannheim analysis, it is necessary that solid and semi-solid foodstuff products be homogenised and dispersed in suitable solvents. For instance water soluble components can be dispersed in water. The solvents preferably should be clear and almost colourless, and therefore cloudy and unclear solutions have to be treated by different method for removing colours and cloudinesses. Fats are filtered, and proteins are precipitated by a Carrez reagent or another precipitation substance. Said method is primarily used for analysis of minor components in foodstuffs like urea, lactic acid, glucose etc.

In food industry NIR (Near Infra Red) spectroscopic methods have grown more and more popular for the reason that said methods provide quick analysis answers, and that they can be used for analysis of solid products with a very little pre-treatment of the product to be tested. The methods require relatively great efforts for maintaining the calibrations of the instruments, and they do not offer a possibility of analysis of all components in the foodstuff with great analysis accuracy.

In the dairy industry there has been used MIR (Mid Infra Red) spectroscopy for analysis of milk since the 1970th. Today the method has an analysis capacity of 500 samples per hour and it makes it possible to analyse most nutritious substances in milk with very great analysis accuracy. The commercial MIR instruments are only capable of analysing liquids. Solid foodstuffs therefore have to be dissolved to provide a homogeneous dispersion for making an analysing by this technique possible. The applications which are present for solid and semi-solid foodstuff analysis are generally complicated and require several manual method steps. As solvent there is used 0.2M NaOH according to the application guide of the instrument supplier (Foss Electric, 1988). In one of the latest application guide of MilkoScan FT 120, Foss Electric Application Note No. 96, September 1996, for hard cheese, the solvent is 0.2M sodium hydroxide with 0.1% Triton X-100. Triton X-100 is a non-ionic detergent (tenside) and is a compound of polyethylene glycol 4-tert-octophanyl-ether. Sodium hydroxide is disadvantageous in that the fat has a tendency of separating, and it is difficult to extract a representative sample.

In another application guide issued by another instrument supplier (Multispec LTD, UK) there is described a method for analysis of cheese, whereby there is added 5 drops of a concentrated washing-up detergent ("Decon 90", UK) to one part of cheese and 5 parts of 0.1 M NaOH ("Multispec", 1988). The product "Decon 90" contains 1-4 % of potassium hydroxide and < 2 % non-ionic of anionic detergents (tensides).

A main purpose with the invention is to suggest a product for dispersing of foodstuffs of all possible types to provide a durable dispersion which is well suited for a quantitative analysis of the contents of the foodstuffs, both as concerns the ingredients and the percentages thereof, by different chemical and physical methods. The content of the dispersing product is of such type that it functions so that the foodstuff is dispersed, and that the solvent, concurrently therewith, has cleaning properties. Thereby it is not necessary to.foresee any washing-up procedures, not even if the prepared solution is used for instance in spectroscopic apparatus.

It has shown that a product containing a mixture of a complexing agent and an alkali (alkali salt) in certain proportions and solved in water, and composed so that a certain pH value is obtained in a solution of the product according to the invention with the product to be analysed, is a very active solvent for all possible types of foodstuffs.

According to the invention which is defined in claim 1, the product consequently is a mixture of a complexing agent, in particular emulsifying salts, and an alkali (alkali salt) in a water solution, in which complexing agent can be present in an amount of 0.1 - 2.0 % of the solution, and the alkali amounts 0.1 - 1.0 % of the solution.

As examples of useful complexing agents in the product according to the invention can be mentioned sodium citrate (Na₃C₆H₅O₇), sodium di-hydrogenphosphate (NaH₂PO₄), di-sodium hydrogenphosphate (Na₂HPO₄), sodium dihydrogen pyrophosphate (Na₂H₂P₂O₇), tri-sodium monohydrogen pyrophosphate (Na₃HP₂O₇), tetra-sodium pyrophosphate (Na₄P₂O₇), penta-sodium triphosphate [old name penta-sodium polyphosphate] (Na₅P₃O₁₀), sodium hexametaphosphate (NaPO₃)ₙ, which can be used separately or in combination with another. In food industry the above complexing agents are named emulsifying salts and the purity is of good grade. It has shown particularly suitable to make use of polyphosphates, for instance phosphates of sodium or potassium, like pentasodium triphosphate.

The alkali (alkali salt) in the product according to the invention is a metasilicate.

The complexing agent and the alkali (alkali salt) are mixed in a proportion of about 2 parts of complexing agent to 1 part of alkali (alkali salt) and are solved in de-ionized water so that the pH value amounts to between pH 10 and pH 13. The content of alkali (alkali salt) preferably can be varied so that the intended pH value is obtained. The foodstuff to be solved in the product solution according to the invention has a certain buffering effect, and therefore the pH value should be adapted with a certain consideration thereto. For best possible results the pH value of the solution of complexing agent and alkali, in which the foodstuff is solved, should be such that the pH value in the dispersion formed by the solution and the foodstuff to be analysed is about 7-11. The water may contain minor amounts or ions if the water is hard, for instance ions of calcium (Ca²⁺) and/or magnesium (Mg²⁺).

The components are known per se and are very common in washing-up substances, in machine washing and in cleaning substances, but, as far as known, no one has so far described a similar product which, according to the invention, can be used for analysis of foodstuffs. As mentioned above it is known to use sodium hydroxide or potassium hydroxide + 5 drops of Decon 90® or 0.1% Triton X-1 00 for dissolving cheese for later use in analysis by spectroscopy. In for instance MIR spectroscopy it has been a common belief that eventual additives should not be allowed to interfere with the analysis. It has therefore been suitable to use sodium hydroxide, since said hydroxide has very little interference at most analytical methods, in particular MIR spectroscopy. By modern computer technology it is today very easy to directly correct for eventual interferences. Complexing agents have been used in the manufacture of processed cheese and are thereby named emulsifying salts or melting salts. The purpose of this process has solely been to give the processed cheese the correct consistency and has not been aimed for analytical purposes.

The product according to the invention, consisting of a combination or a complexing agent and an alkali (alkali salt) can be supplied as a ready powder mixture or in concentrated form and mixed in suitable proportions. At the manufacture or the solvent for the foodstuff the mixed powder is solved in de-ionized water, for instance in an amount of combined powder of 0.1-4 % and the remaining part being de-ionized water, or to the concentrate is added water so that the correct final concentration is obtained in the solution to be used. The temperature should be about 40°C, both when the product powder is dissolved in water. To the prepared product solution is added the product to be analyzed in an amount of 10 % by weight of foodstuff to 90 % by weight of the product solution. The mixture is finely ground in a mill of suitable type, whereby a durable dispersion is obtained within the course of 20-300 seconds. The solution is shaken and has the appearance of a milk white dispersion, which in practice generally is a combination of a dispersion and micro particles. The pH value should, as mentioned above, be about 7-11.

The analysis of the dispersion is made as known per se in that the dispersion is introduced in an IR-spectroscopy-analysis apparatus of known type, whereby an analysis result is directly obtained presenting the existing components of the foodstuff and the percentual amounts thereof. For foodstuffs there is generally requested information about fat, proteins, carbohydrates, organic acids like lactic acid, acetic acid, butyric acid, etc., and the content of water in the product.

### EXAMPLE 1

For analysis of a foodstuff product, in this case cheese, there were performed a great number of tests.

For the analysis of cheese there was mixed 6 g of a powder of pentasodium triphosphate (Na₅P₃O₁₀) with 2.5 g of a powder of disodium metasilicate (Na₂Si₂O₃) to a powder batch suited for being solved in de-ionized water to a total volume of 1 litre.

The product powder was dissolved in de-ionized water of 40°C to a total volume of 1 litre. The pH value of the product solution was about 11.7. To said product solution was added pieces of different types of hard cheese in an amount by weight corresponding to 10 % by weight and 90 % by weight of the product solution. The mixture of the solution and cheese was introduced in a mill of the type LOSMIXER (manufactured by L O Sjaunja AB, Uppsala, Sweden), which after 40-90 seconds presented a milk like liquid in the form of a dispersion having a pH value, buffered by the cheese, of 7-11. The dispersion which was obtained, which might actually have been analyzed by any chemical or physical method, was in this case analyzed by Mid Infra Red (MIR) transmission spectroscopy of the type DAIRYLAB (Multispec Ltd, York, UK).

The accuracy of the analysis, given as a standard deviation (SD) of the difference between MIR and the chemical official reference methods according to IDF (International Dairy Federation) was very good. In the following table the analysis accuracy is shown.

**TABLE 1**

| **COMPOSITION** | | | | | **ANALYSIS ACCURACY** | |
|---|---|---|---|---|---|---|
| Parameter | Number | Means | Min. | Max. | SD | RSD¹⁾ |
| Fat % | 64 | 26.5 | 16.2 | 39.0 | 0.18 | 0.68 |
| Protein % | 64 | 25.3 | 19.7 | 30.3 | 0.21 | 0.83 |
| Lactose % | 64 | 0.22 | 0.02 | 0.40 | 0.04 | 18.0 |
| Dry matter % | 64 | 57.4 | 52.5 | 63.7 | 0.43 | 0.75 |
| Lactic acid % | 64 | 1.25 | 0.85 | 1.52 | 0.03 | 2.40 |

| | | | | | | |
|---|---|---|---|---|---|---|
| 1) RSD = relative standard deviation (SD * 100)/mean value | | | | | | |

### FURTHER EXAMPLES

In the same way as referred to above and with different proportions of complexing agent and alkali and with a pH value of between 7 and 11 Example 1 was repeated based on fish, meat, ready food, flower and grain products. Comparative chemical analysis were performed for verifying the results which were obtained.

It was shown that the product according to the invention, consisting of different parts of complexing agent and alkali, in total about 0.1-4 % by weight of the powder mixture, solved in water, was capable of dissolving the various foodstuffs to provide an even and smooth dispersion, milled in a mill of known type during the course of 20-300 seconds.

The accuracy of the analysis was very good and the analysis results in the form of the contents of fat, proteins, carbohydrates, organic acids, water and eventually existing foreign components were quickly and simply obtained, with a great accuracy of analysis, by means of known analysis equipment.

## Claims

1. A product capable of providing a durable dispersion of a foodstuff, when said foodstuff is finely ground in a mill together with a liquid and the product,
**characterized in that**
it consists of a powder mixture of a complexing agent and an alkali, wherein said complexing agent is a polyphosphate, selected from phosphates of sodium or potassium, suitably penta-sodium-tri-phosphate, and wherein
the alkali is meta-silicate.

2. A product as claimed in claim 1, wherein the proportion between alkali and complexing agent is about 1:2.

3. A product as claimed in any preceding claim, the composition of which is such that a foodstuff sample preparation comprising 0,1 - 4% of the product, 10% of the foodstuff, and the balance water, based on the total weight of the preparation, obtains a pH in the interval 7-11.

4. Solution of a product as claimed in any of claims 1-3 dissolved in water.

5. Solution as claimed in claim 4 the pH of which is between 10 and 13.

## Patentansprüche

1. Ein Produkt, das in der Lage ist eine dauerhafte Dispergierung eines Nahrungsmittel bereitzustellen, wenn das Nahrungsmittel in einer Mühle zusammen mit einer Flüssigkeit und dem Produkt fein gemahlen wird,
**dadurch gekennzeichnet, dass**
es aus einem Pulvergemisch von einem Komplexbildner und einem Alkali besteht, wobei der Komplexbildner ein Polyphosphat ist, ausgewählt aus Phosphaten von Natrium oder Kalium, geeigneterweise Pentanatriumtriphosphat, und wobei
das Alkali ein Metasilikat ist.

2. Ein Produkt wie in Anspruch 1 beansprucht, wobei das Verhältnis zwischen Alkali und Komplexbildner etwa 1:2 ist.

3. Ein Produkt wie in einem der vorhergehenden Ansprüche beansprucht, dessen Zusammensetzung derart ist, dass eine Nahrungsmittelprobenpreparation, die 0,1 bis 4 % des Produkts, 10 % des Nahrungsmittels, und das Ausgleichswasser, basierend auf dem Gesamtgewicht der Präparation umfasst, einen pII-Wert in dem Intervall 7 bis 11 aufweist.

4. Lösung eines Produkts wie in einem der Ansprüche 1 bis 3 beansprucht, gelöst in Wasser.

5. Lösung wie in Anspruch 4 beansprucht, deren pH zwischen 10 und 13 liegt.

## Revendications

1. Produit capable de provoquer une disporsion durable d'une denrée alimentaire lorsque ladite denrée alimentaire est finement broyée dans un broyeur conjointement avec un liquide et le produit,
**caractérisé en ce que**
il consiste en un mélange en poudre d'un agent complexant et d'un agent alcalin, dans lequel lodit agent complexant est un polyphosphate choisi entre des phosphates de sodium ou de potassium, convenablement le triphosphate pentasodique, et dans lequel
J.'aq;nt alcalin est un métasilicate.

2. Produit suivant la revendication 1, dans lequel le rapport de l'agent alcalin à l'agent complexant est égal à environ 1:2.

3. Produit suivant l'une quelconque des revendications précédentes, dont la composition est telle qu'une préparation d'un échantillon de denrée alimentaire Comprenant 0,1 à 4 % du produit, J 0 % de la denrée alimentaire et le pourcentage restant d'eau, sur la base du poids total de la préparation, atteint un pil dans l'intervalle de 7 à 11.

4. Solution d'un produit suivant l'une quelconque des revendications 1 à 3 dissous dans de l'eau.

5. Solution suivant la revendication 4, dont le pH est compris entre 10 et 13.
